Europäisches Patentamt

European Patent Office

Office européen des brevets

(19)

(11) Veröffentlichungsnummer: **0 141 343**
**B1**

(12) **EUROPÄISCHE PATENTSCHRIFT**

(45) Veröffentlichungstag der Patentschrift:
**01.06.88**

(21) Anmeldenummer: **84112547.9**

(22) Anmeldetag: **18.10.84**

(51) Int. Cl.⁴: **G 01 N 33/92, C 12 Q 1/60**

(54) Verfahren zur Bestimmung der Low Density Lipoproteine (LDL) und Reagenz zu seiner Durchführung.

(30) Priorität: **26.10.83 DE 3338836**

(43) Veröffentlichungstag der Anmeldung:
**15.05.85 Patentblatt 85/20**

(45) Bekanntmachung des Hinweises auf die Patenterteilung:
**01.06.88 Patentblatt 88/22**

(84) Benannte Vertragsstaaten:
**AT BE CH DE FR GB IT LI LU NL SE**

(56) Entgegenhaltungen:
**EP - A - 0 092 801
DE - A - 2 612 725
FR - A - 2 455 743
US - A - 3 884 764**

**CLINICAL CHEMISTRY, Band 28, Nr. 6, Juni 1982, Seiten 1379-1388, Washington, US; G.R. WARNICK et al.: "Dextran sulfate-Mg2+ precipitation procedure for quantitation of high-density-lipoprotein cholesterol"**

(73) Patentinhaber: **Boehringer Mannheim GmbH, Sandhofer Strasse 116, D-6800 Mannheim 31 (DE)**

(72) Erfinder: **Kerscher, Lorenz, Dr. rer. nat., Hanfelder Strasse 24 a, D-8130 Starnberg (DE)**
Erfinder: **Ziegenhorn, Joachim, Dr. rer. nat., Ina-Seidel-Weg 1, D-8130 Starnberg (DE)**
Erfinder: **Schiefer, Sigbert, Dr. rer. nat., Moosstrasse 3, D-8121 Pähl (DE)**

## Beschreibung

Die Erfindung betrifft ein Verfahren und ein Reagenz zur Bestimmung der Low Density Lipoproteine (LDL).

Die Bestimmung der LDL-Fraktion (Low Density Lipoproteine), auch β-Lipoproteinfraktion genannt, hat für die differenzierte Diagnose einer Lipidstoffwechselstörung erhebliche Bedeutung erlangt.

Hypercholesterinämie und Hypertriglyceridämie begünstigen die Entstehung der Atherosklerose und des Herzinfarkts. Die Bestimmung von Cholesterin und Triglyceriden im Serum gehören daher zu den am häufigsten durchgeführten Tests im klinischchemischen Routinelabor.

Zahlreiche Untersuchungen zum Fettstoffwechsel kommen zu dem Schluß, daß das individuelle Coronarrisiko besser erfaßt werden kann, wenn nicht nur die Veränderung im Triglycerid- und Cholesterinspiegel bestimmt, sondern die zugrundeliegenden pathologischen Verschiebungen im Lipoproteinmuster ermittelt werden (Münch. med. Wschr. 121, [1979] 1639).

Die bekannten Plasmalipoproteine enthalten einen unterschiedlichen hohen Anteil an Protein (Apolipoproteine), Phospholipiden, Cholesterin und Triglyceriden. Sie lassen sich aufgrund ihres Verhaltens (unterschiedliche Dichte) in der analytischen Ultrazentrifuge und aufgrund ihrer unterschiedlichen Wanderungsgeschwindigkeit in der Gel-Elektrophorese in vier verschiedene Klassen unterteilen:

Chylomikronen
Prä-β-Lipoprotein = VLDL (Very Low Density Lipoprotein)
β-Lipoprotein      = LDL (Low Density Lipoprotein)
α-Lipoprotein      = HDL (High Density Lipoprotein)

Die Untersuchung der Funktion der Lipoproteine ergab, daß LDL innerhalb der Lipoproteine die entscheidende atherogene Komponente darstellt, bei deren Erhöhung im Blut ein gesteigertes Risiko für eine coronare Herzkrankheit vorliegt. Die frühzeitige Erkennung und Bekämpfung dieses Zustands ist von großer Bedeutung. Es besteht daher ein Bedarf nach einem praktikablen Verfahren zur quantitativen Bestimmung der LDL-Konzentration im Serum und Plasma.

Bisher wurden für die Bestimmung des LDL-Cholesterinwerts im wesentlichen drei Methoden eingesetzt, die jedoch Nachteile besitzen:

1. Ultrazentrifugation. Dieses Verfahren ist für ein Routinelabor nicht geeignet, da man hierzu eine spezielle Geräteausrüstung benötigt und die Durchführung eine äußerst sorgfältige Arbeitstechnik und einen sehr hohen Zeitaufwand (mehrtägiges Zentrifugieren) in einer Ultrazentrifuge erfordert. Somit blieb dieses Analysenverfahren bisher nur dem Labor der forschenden Medizin vorbehalten.

2. Elektrophoretische Trennung mit anschließender Visualisierung der Lipoproteinbanden durch Polyanionenfällung und Umrechnung der Trübungseinheiten in Cholesterinwerte.

Dieses Verfahren jedoch ist zeitaufwendig und erfordert eine eigene Elektrophorese-Apparatur sowie ein Densitometer zur Auswertung (Lab. Med. 1 [1977] 145).

3. Bestimmung des LDL-Cholesterin-Wertes über die Friedewald-Formel (Clin. Chem. 18 [1972] 499).

Für die Errechnung des LDL-Cholesterin-Wertes nach der Friedewald-Formel ist die Bestimmung von 3 Parametern notwendig: Cholesterin-, HDL-Cholesterin- und Triglyceridwert der Probe. Die Methode ist damit nicht ausreichend praktikabel. Außerdem gilt diese Nährungsformel nur für chylomikronenfreie Proben und Proben mit Triglyceridwerten unter 400 mg/dl.

4. Fällungsreaktionen. Ein Verfahren, bei welchem LDL mit Hilfe eines Lectins präzipitiert wird, ist in der DE-OS 28 57 710 beschrieben. Jedoch kann bei dieser Methode der Wert für LDL-Cholesterin erst nach Wiederauflösen des Präzipitats bzw. nur durch Differenzbildung der Cholesterinwerte vor und nach Präzipitation ermittelt werden. Dies stellt einen erheblichen Nachteil dar.

Eine Präzipitationsmethode für Lipoproteine, bei welcher LDL im Überstand der Fällung verbleibt, wird in der DE-PS 26 00 664 beschrieben. Die Methode ist jedoch für die Anwendung als Routinebestimmung nicht ausreichend praktikabel, da für die Ausfällung der Lipoproteine 2 Arbeitsschritte erforderlich sind (Zugabe zweier unterschiedlicher Agentien – Polyäthylenimin und ein Kationenaustauscher – verbunden mit einer dazwischenliegenden Inkubationsphase).

Es besteht daher ein Bedarf nach einem einfachen Verfahren und Reagenz mit hoher Praktikabilität und großer Treffsicherheit für die Bestimmung von LDL-Lipoprotein. Nach EP-A-92801 ist dieses Problem gelöst worden durch ein Verfahren zur Bestimmung von Low Density Lipoproteine (LDL) in Körperflüssigkeiten, welches dadurch gekennzeichnet ist, daß man der zu untersuchenden Probe High-Density-Lipoprotein (HDL)-Antikörper zusetzt, gebildetes Unlösliches abtrennt und im Überstand das LDL oder eine seiner Komponenten bestimmt.

Es hat sich gezeigt, daß es vorteilhaft wäre, wenn besonders bei hyperlipidämischen, d.h. VLDL-reichen Seren die Bildung des Immunpräzipitats beschleunigt und die Immunkomplexe noch einfacher vollständig abzentrifugiert werden könnten.

Diese Aufgabe wird erfindungsgemäß gelöst dadurch, daß man dem Antikörper ein Gemisch aus einem Polyanion und einem zweiwertigen Kation zusetzt, wobei die Polyanionen in einer Konzentration von 0,1–15 g/l, die zweiwertigen Kationen in einer Konzentration von 10–250 mmol/l im Reaktionsgemisch mit einer Gesamtsalzkonzentration, die höher als 150 mmol/l ist, vorliegen.

Die Erfindung beruht auf der überraschenden Feststellung, daß durch den Zusatz des Gemisches aus Polyanionen und zweiwertigen Kationen in den angegebenen Konzentrationen die Antikörpereigenschaften nicht nachteilig beeinflußt werden. Bisher war außerdem zu befürchten, daß durch die Zugabe von Polyanion nicht nur die gewünschte Ausfällung von VLDL und anderen Lipoproteinfraktionen, sondern auch die Ausfällung des LDL, welches ja zu bestimmen ist, herbeiführt. Überraschenderweise wird aber bei Salzkonzentrationen, die höher als etwa 150 mmol/l sind, die schnelle und vollständige Aggregation der Lipoprotein-Antikörper-Komplexe durch die Anwesenheit der Polyanionen und zweiwertigen Kationen begünstigt und deren Zentrifugierbarkeit erhöht, ohne daß es zu einer Mitfällung der LDL kommt. Als vorteilhaft hat sich die Verwendung von Polyanionen wie Dextransulfat, Heparin, Phosphorwolframsäure und Polyvinylsulfat und von Chloriden zweiwertiger Kationen wie Calcium-, Magnesium- und Manganchlorid erwiesen. Das Dextransulfat kann hochmolekular (Molekulargewicht $5 \times 10^4 - 2 \times 10^6$) oder kurzkettig (Molekulargewicht 5000–50 000) sein. Es kann von besonderem Vorteil sein, die Antikörper kovalent an Dextransulfat oder Heparin zu binden, wobei die Verknüpfung nach üblichen Methoden erfolgt und die so erhaltenen Konjugate zusammen mit Calcium-, Magnesium- oder Manganchlorid in den Test einzusetzen.

Die bevorzugten Konzentrationsbereiche der Polyanionen im Reaktionsgemisch sind 0,1–8 g/l beim hochmolekularen Dextransulfat, 1–15 g/l beim kurzkettigen Dextransulfat und dem Heparin, 0,2–5 g/l beim Polyvinylsulfat und 0,3–6 g/l bei der Phosphorwolframsäure. Die Konzentration der einzusetzenden zweiwertigen Metallionen beträgt 10–250 mmol/l im Reaktionsgemisch. Weiterhin enthält das Reaktionsgemisch bevorzugt Natriumchlorid in einer Konzentration von 0,1–1 mol/l. Als einzusetzende Puffer kommen übliche Pufferlösungen im pH-Bereich von etwa 6,5–8,5 in Frage, wie MES (Morpholinäthansulfonsäure.), Triethanolamin, MOPS (Morpholinopropansulfonsäure.), Tris-Puffer. Die Antikörper liegen bevorzugt in der Konzentration entsprechend 1–100 mg/ml γ-Globulin vor.

Die im Überstand des Reagenz verbliebene LDL-Fraktion kann dann nach den hierfür üblichen Methoden bestimmt werden. Bevorzugt erfolgt die Bestimmung des darin enthaltenen gebundenen Cholesterins unter Anwendung der hierfür bekannten Methoden. So kann die Bestimmung beispielsweise durch Verseifung mit alkoholischer Kalilauge und chemische Bestimmung nach Liebermann-Burchard durchgeführt werden. Bevorzugt erfolgt jedoch eine enzymatische Bestimmung unter Verwendung von Cholesterinoxidase und einem Cholesterinester spaltenden Enzym oder Enzymsystem, wie insbesondere Cholesterinesterase. Bei Anwendung der letzteren Methode kann verbrauchter Sauerstoff, gebildetes Cholestenon oder, am meisten bevorzugt, gebildetes $H_2O_2$ nach den dem Fachmann hierfür bekannten Methoden bestimmt werden. Da die Bestimmung des gebundenen Cholesterins dem Fachmann bekannt ist, braucht hierauf im vorliegenden Zusammenhang nicht näher eingegangen zu werden. Es sei jedoch bemerkt, daß im Rahmen des erfindungsgemäßen Verfahrens durch die Entfernung der VLDL- und Chylomikronen-Fraktionen das Auftreten von Trübungen verhindert wird, welche eine optische Messung von Cholestenon oder $H_2O_2$ im Rahmen von Farbreaktionen stören könnten. Das Verfahren eignet sich daher in besonderem Maße in Verbindung mit einer colorimetrischen Cholesterinbestimmungsmethode.

Es ist jedoch auch möglich, anstelle des in der LDL-Fraktion enthaltenen Cholesterins bzw. anderer LDL-Komponenten wie Apolipoprotein B, Phospholipide und Triglyceride die LDL-Fraktion selbst zu bestimmen, wobei ebenfalls an sich bekannte Methoden angewendet werden können. Beispielsweise genannt sei die nephelometrische Bestimmung oder die in der DE-OS 30 07 764 beschriebene turbidimetrische Bestimmung.

Die HDL-Antikörper werden im Rahmen der Erfindung bevorzugt entweder in Form eines entfetteten HDL-Antiserums oder in Form von daraus gereinigten HDL-Antikörperfraktionen verwendet. Es ist auch möglich, HDL-Antikörperfragmente, beispielsweise Fab, Fab₂ und Fab'-Fragmente zu verwenden. Ebenso ist es möglich, Antikörper gegen die Apolipoproteine A, C oder/und E des HDL oder deren Fragmente zu verwenden. Schließlich können auch monoklonale HDL-Antikörper eingesetzt werden.

Die Herstellung der erfindungsgemäß verwendeten Antikörper erfolgt mit Verwendung von reinem HDL oder einem der genannten Apolipoproteine als Immunogen. Die so gewonnenen Antiseren werden durch Behandlung mit Aerosil® delipidiert; gegebenenfalls wird anschliessend durch Ammoniumsulfat-Fraktionierung die γ-Globulin-Fraktion isoliert.

Für die Antikörpergewinnung können die üblicherweise verwendeten Tierspezies herangezogen werden. Bevorzugt werden Schaf und Kaninchen. Für die Antikörpergewinnung können aber außer den schon erwähnten Tieren bzw. vergleichbaren Lebewesen auch Zellkulturen verwendet werden.

Die Abtrennung der beim Zusatz der HDL-Antikörper gebildeten Immunaggregate kann ebenfalls nach üblichen Methoden erfolgen. Werden lösliche HDL-Antikörper verwendet, so erfolgt die Abtrennung zweckmäßig durch Zentrifugieren. Werden immobilisierte, trägergebundene HDL-Antikörper eingesetzt, so lassen sich die Immunaggregate durch einfache Trennung der flüssigen Phase von der kompakten festen Phase, beispielsweise einem mit Antikörper beschichteten Festkörper, trennen. Verwendet man mit Apolipoproteinen als Immunogen gewonnene Antikörper, so werden vorzugsweise solche verwendet, bei denen das als Immunogen verwendete Apolipoprotein eine Lipidumhüllung aufweist. Dies läßt sich beispielsweise erreichen, indem man nach der üblichen Delipidierung und anschließenden Fraktionierung der Apolipoproteine die ausgewählte Apolipoprotein A-, C- oder/und E-Fraktion wieder relipidiert. Bevorzugt verwendet man als Immunogen jedoch gereinigte vollständige HDL-Fraktion. Die Reinigung erfolgt zweckmäßig in an sich bekannter Weise durch Isolierung in der Ultrazentrifuge. Zusätzlich kann gegebenenfalls eine weitere Reinigung über z.B. immobilisiertes concanavalin A nach den Methoden der Affinitätschromatographie oder der Elektrophorese vorgenommen werden. Diese Methoden sind dem Fachmann bekannt und brauchen hier nicht näher beschrieben werden.

Ein weiterer Gegenstand der Erfindung ist ein Reagenz zur Bestimmung der LDL-Fraktion von Körperflüssigkeiten, welches durch einen Gehalt an HDL-Antikörpern gekennzeichnet ist. In einer bevorzugten Ausführungsform enthält das erfindungsgemäße Reagenz neben den schon erwähnten HDL-Antikörpern bzw. den oben bei der Erläuterung des Verfahrens näher beschriebenen Fragmenten oder Antikörpern bzw. Fragmenten von HDL-Komponenten noch ein Reagenz zur Bestimmung von Cholesterin.

Ein bevorzugtes Reagenz der vorstehend genannten Art enthält Cholesterinoxidase, ein Cholesterinester spaltendes Enzym oder Enzymsystem, ein System zur Bestimmung von $H_2O_2$ und ein oberflächenaktives Mittel.

Gemäß einer besonders bevorzugten Ausführungsform des vorstehend erwähnten Reagenz besteht dieses im wesentlichen aus HDL-Antikörper, Cholesterinoxidase, Cholesterinesterase, Peroxidase, 3,4-Dichlorphenol, Phenol, 4-Aminophenazon, einem nichtionischen Detergenz, Magnesiumaspartat und Puffer pH 7 bis 8,5.

Das erfindungsgemäße Reagenz enthält den Antikörper zweckmäßig im Konzentrationsbereich von $10^{-7}$ bis $10^{-3}$ mol/l (bzw. Kilo Festkörper), bezogen auf die Bestimmungslösung. Der Antikörper kann in fester Form, vorzugsweise lyophilisiert, oder in Form einer Lösung vorliegen. Als Lösungsmittel können Serummilieu, Puffer wie z. B. 0,01 bis 0,5 m Trispuffer, pH 7 bis 8,5 oder 0,01 bis 0,5 m Morpholinopropansulfonsäure pH 6,5–7,5, jeweils unter Zusatz von 0,15–1 m Kochsalz verwendet werden. Wird der Antikörper in immobilisierter Form verwendet, so sind Beispiele für geeignete Trägersubstanzen Polysaccharide wie Cellulose, Dextran, Stärke und deren Derivate, Silikate, Polyamide, Kollagen, Latex, Aluminiumoxid, Rinderserumalbumin und ähnliche Trägersubstanzen. Der Antikörper kann auch an der Oberfläche von Testbehältern, wie Kunststoffreagenz-Gläsern, gebunden vorliegen.

Ein wesentlicher Vorteil des erfindungsgemäßen Verfahrens liegt darin, daß sich nach Zugabe eines einzigen Reagenzes die Lipoproteinklassen – mit Ausnahme von LDL – aus der Probe entfernen lassen und der diagnostisch bedeutsame LDL-Gehalt oder der Cholesteringehalt der LDL-Fraktion anschließend ohne weitere Probenvorbehandlung einer direkten Messung zugänglich ist. Vorteilhaft ist weiter, daß die in der Probe Trübungen verursachenden triglyceridreichen Lipoproteinklassen entfernt werden, so daß zur anschließenden LDL- oder Cholesterinbestimmung eine klare Probe zur Verfügung steht.

Die folgenden Beispiele erläutern die Erfindung anhand einer bevorzugten Ausführungsform.

Beispiel 1

A) Herstellung von gereinigter HDL.
Nach Abtrennung von VLDL und LDL in der Ultrazentrifuge wird eine eng geschnittene HDL-Fraktion (d 1,080 bis 1,210) in der Ultrazentrifuge isoliert, wie bei V.P. Skipski: Lipid composition of lipoproteins in normal and diseased states, in: Blood Lipids and Lipoproteins: Quantitation, Composition and Metabolism. Hrsg.: Nelson, Wiley, New York 1972, 471–583 beschrieben. Die Fraktion wird anschließend noch zweimal bei den Dichten 1,080 und 1,210 sedimentiert bzw. flotiert.

Die HDL-Fraktion wird über immobilisiertes Concanavalin A ([1974] Febs Lett. 91, 174–198) affinitätschromatographisch oder mittels Geon-Pevicon-Block-Elektrophorese nach R.W. Mahley, K.S. Holcombe (1977), J. Lipid. Res. 18, 314–324, elektrophoretisch gereinigt.

B) Herstellung des Antiserums.
Tierspecies: Schaf oder Kaninchen
Unter Verwendung des nach A erhaltenen Immunogens wird folgendes Immunisierungsschema angewendet:

| Tag | Applikation | Immunogenmenge |
|---|---|---|
| 0 | intradermal | 1 mg Protein (HDL) emulgiert in Freund Adjuvans |
| 7 | intramuskulär | 1 mg Protein (HDL) emulgiert in Freund Adjuvans |
| 14 | subcutan | 1 mg Protein (HDL) emulgiert in Freund Adjuvans |
| 30 | intramuskulär | 1 mg Protein (HDL) emulgiert in Freund Adjuvans |
| 60 | subcutan | 1 mg Protein (HDL) emulgiert in Freund Adjuvans |
| alle weiteren 30 Tage | subcutan | 1 mg Protein (HDL) emulgiert in Freund Adjuvans |

Die erste Probeblutung wird nach 45 Tagen vorgenommen.

C) Herstellung von anti-HDL-γ-Globulin (delipidiert). 1 l Rohserum wird mit 15 g Aerosil® 380 (Degussa) 1 Std. bei Raumtemperatur gerührt und anschließend 20 min bei 5000 g zentrifugiert. Der Überstand wird bis zu einer Endkonzentration von 1,7 mol/l mit Ammoniumsulfat versetzt und 2 Std. gerührt. Nach 30 min Zentrifugation bei 20 000 g wird der Niederschlag in 200 ml phosphatgepufferter (pH 7,4) physiologischer Kochsalzlösung gelöst und durch Dialyse völlig vom Ammonsulfat getrennt. Durch 2 Std. Zentrifugation bei 50 000 g wird die so erhaltene γ-Globulin-Fraktion von unlöslichem Protein befreit.

Beispiel 2
Zu 0,4 ml eines Gemisches aus 1 g/l Dextransulfat (mittleres Molekulargewicht 500 000), 0,3 mol/l Natriumchlorid, 50 mmol/l Calciumchlorid und 15 mg anti-HDL-γ-Globulin (delipidiert), welches wie in Beispiel 1 beschrieben gewonnen wurde, in 0,1 mol/l Tris/HCl-Puffer vom pH-Wert 8,0 gibt man 30 μl Serumprobe. Nach 30minütiger Inkubation bei Raumtemperatur wird 2 Minuten bei 10 000 g zentrifugiert. Entsprechende Kontrollversuche unter Weglassung von Dextransulfat und Calciumchlorid wurden parallel unter entsprechenden Bedingungen durchgeführt.

300 μl des klaren Präzipitationsüberstands werden mit 2 ml eines Reagenzes versetzt, das 0,1 mol/l Tris-Puffer, pH = 7,7; 0,05 mol/l Magnesiumaspartat; 1 mmol/l 4-Aminophenazon, 6 mmol/l Phenol,

4 mmol/l 3,4-Dichlorphenol, 0,3% Fettalkoholpolyglycoläther, 400 U/l Cholesterinesterase, 250 U/l Cholesterinoxidase und 200 U/l Peroxidase enthält.

Nach 20 Minuten Inkubationszeit bei Raumtemperatur wird die Extinktion der Probe gegen den Reagenzienleerwert gemessen (der Reagenzienleerwert enthält 50 µl des Antiserums und berücksichtigt den Cholesteringehalt des Antiserums).

$$\Delta E = \Delta E_{Probe} - \Delta E_{Reagenzienleerwert}$$
$$LDL-Cholesterin\ (mg/dl) = 894 \times \Delta E$$

Es wurden folgende Ergebnisse erhalten:

| Serum | Gesamt-Triglyceride (mg/dl) | Referenzmethode NIH-Verfahren* | | Immunpräz. inkl. Dextransulf. + Ca²⁺ | | Immunpräz. ohne Dextransulf. + Ca²⁺ | |
|---|---|---|---|---|---|---|---|
| | | LDL-Chol (mg/dl) | LDL-TG (mg/dl) | LDL-Chol (mg/dl) | LDL-TG (mg/dl) | LDL-Chol (mg/dl) | LDL-TG (mg/dl) |
| 1 | 1049 | 89 | 52 | 91 | 78 | 154 | 471 |
| 2 | 333 | 143 | 69 | 128 | 83 | 158 | 175 |
| 3 | 227 | 137 | 61 | 134 | 56 | 141 | 126 |
| 4 | 806 | 156 | 46 | 141 | 52 | 187 | 388 |
| 5 | 231 | 133 | 44 | 129 | 49 | 135 | 114 |
| 6 | 218 | 158 | 64 | 159 | 72 | 198 | 146 |

\* Als Referenzmethode dient das NIH-Verfahren (nach Abtrennung von VLDL und Chylomikronen in der Ultrazentrifuge wird LDL präzipitiert. Aus der Differenz der Cholesterinwerte vor und nach Fällung erhält man den Wert für LDL-Cholesterin). Manual of Laboratory Operations, Lipid Research Clinics Program, Lipid and Lipoprotein Analysis, DHEW Publication No. 65–628.

Vergleichbare Resultate erhält man mit 1 g/l Dextransulfat (Molekulargewicht 2 000 000). Statt Calciumionen kann man auch Magnesiumionen oder Manganionen in gleicher Konzentration verwenden. Bevorzugt sind folgende Konzentrationsbereiche: Hochmolekulares Dextransulfat 0,1–8 g/l, zweiwertiges Kation 10–250 mmol/l, Natriumchlorid 0,2–1 mol/l.

Beispiel 3

Zu 0,4 ml eines Gemisches von 5 g/l kurzkettigem Dextransulfat (mittleres Molekulargewicht 5000), 0,25 mol/l Natriumchlorid, 0,15 mol/l Calciumchlorid, und 15 mg anti-HDL-γ-Globulin (delipidiert) in 0,1 mol/l Tris/HCl-Puffer von pH 8,0 werden 30 µl Serumprobe gegeben. Nach 30minütiger Inkubation bei Raumtemperatur wird 2 Minuten bei 10 000 g zentrifugiert und Cholesterin sowie Triglyceride im Überstand gemessen, nach der Methode wie in Beispiel 1 unter C beschrieben. Ensprechende Kontrollversuche wurden parallel unter entsprechenden Bedingungen bei Weglassung von Dextransulfat und Calciumionen durchgeführt. Es wurden folgende Ergebnisse erhalten:

| Serum | Gesamt-Triglyceride (mg/dl) | Referenzmethode NIH-Verfahren | | Immunpräz. inkl. Dextransulf. + Ca²⁺ | | Immunpräz. ohne Dextransulf. + Ca²⁺ | |
|---|---|---|---|---|---|---|---|
| | | LDL-Chol (mg/dl) | LDL-TG (mg/dl) | LDL-Chol (mg/dl) | LDL-TG (mg/dl) | LDL-Chol (mg/dl) | LDL-TG (mg/dl) |
| 1 | 329 | 158 | 54 | 154 | 68 | 170 | 210 |
| 2 | 344 | 314 | 89 | 301 | 104 | 362 | 209 |
| 3 | 512 | 246 | 79 | 236 | 85 | 265 | 268 |
| 4 | 736 | 171 | 80 | 174 | 65 | 241 | 312 |
| 5 | 197 | 135 | 102 | 137 | 85 | 134 | 119 |
| 6 | 668 | 157 | 75 | 142 | 89 | 199 | 343 |

Vergleichbare Resultate erhält man mit 0,5 g/l Dextransulfat (Molekulargewicht 15 000), 0,8 g/l Heparin oder 0,2 g/l Polyvinylsulfat. Statt Calciumionen können auch Magnesiumionen oder Manganionen in entsprechender Konzentration verwendet werden. Vorteilhafte Konzentrationsbereiche sind: Kurzkettiges Dextransulfat bzw. Heparin 1–15 g/l, Polyvinylsulfat 0,2–5 g/l, zweiwertiges Kation 20–250 mmol/l, Natriumchlorid 0,15–0,8 mol/l.

Beispiel 4

Zu 0,4 ml eines Gemisches von 1,15 g/l Phosphowolframsäure, 20 mmol/l Magnesiumchlorid, 0,3 mol/l Natriumchlorid und 15 mg anti-HDL-γ-Globulin (delipidiert) werden 30 µl Serumprobe gegeben. Nach 30minütiger Inkubation bei Raumtemperatur wird 2 Minuten bei 10 000 g zentrifugiert und Cholesterin sowie Triglyceride im Überstand gemessen. Es wurden parallel Kontrollversuche unter Weglassung von Dextransulfat und Calciumchlorid durchgeführt. Es wurden folgende Ergebnisse erhalten:

| Serum | Gesamt-Triglyceride (mg/dl) | Referenzmethode NIH-Verfahren | | Immunpräz. inkl. PWS/Mg²⁺ | | Immunpräz. ohne Zusatz | |
|---|---|---|---|---|---|---|---|
| | | LDL-Chol (mg/dl) | LDL-TG (mg/dl) | LDL-Chol (mg/dl) | LDL-TG (mg/dl) | LDL-Chol (mg/dl) | LDL-TG (mg/dl) |
| 1 | 329 | 158 | 54 | 150 | 57 | 170 | 270 |
| 2 | 344 | 314 | 89 | 299 | 99 | 362 | 209 |
| 3 | 512 | 246 | 79 | 241 | 83 | 265 | 268 |
| 4 | 736 | 171 | 80 | 164 | 72 | 249 | 312 |
| 5 | 147 | 135 | 102 | 131 | 83 | 134 | 119 |
| 6 | 668 | 157 | 75 | 153 | 78 | 199 | 343 |

Die vorteilhaften Konzentrationsbereiche sind: Phosphowolframsäure 0,3–6 g/l, Magnesiumionen 10–200 mmol/l.

## Patentansprüche

1. Verfahren zur Bestimmung der Low Density Lipoproteine (LDL) in Körperflüssigkeiten, bei dem man der zu untersuchenden Probe High-Density-Lipoprotein (HDL)-Antikörper zusetzt, gebildetes Unlösliches abtrennt und im Überstand das LDL oder eine seiner Komponenten bestimmt, dadurch gekennzeichnet, daß man dem Antikörper ein Gemisch aus einem Polyanion und einem zweiwertigen Kation zusetzt, wobei die Polyanionen in einer Konzentration von 0,1–15 g/l, die zweiwertigen Kationen in einer Konzentration von 10–250 mmol/l im Reaktionsgemisch mit einer Gesamtsalzkonzentration höher als 150 mmol/l vorliegen.

2. Verfahren nach Anspruch 1, dadurch gekennzeichnet, daß man als Polyanionen Dextransulfat, Heparin, Phosphowolframsäure oder Polyvinylsulfat und als zweiwertige Kationen Calcium-, Magnesium- oder Mangan-Ionen zusetzt.

3. Verfahren nach einem der Ansprüche 1 oder 2, dadurch gekennzeichnet, daß die Konzentration bei hochmolekularem Dextransulfat 0,1–8 g/l, beim kurzkettigen Dextransulfat 1–15 g/l, beim Heparin 1–15 g/l, beim Polyvinylsulfat 0,2–5 g/l, bei der Phosphorwolframsäure 0,3–6 g/l, bei den zweiwertigen Kationen 10–250 mmol/l im Reaktionsgemisch betragen.

4. Verfahren nach einem der Ansprüche 1–3, dadurch gekennzeichnet, daß man als LDL-Komponente Cholesterin bestimmt.

5. Verfahren nach einem der Ansprüche 1–4, dadurch gekennzeichnet, daß der Antikörper in Form von entfettetem HDL-Antiserum oder als gereinigte Antikörperfraktion vorliegt.

6. Verfahren nach einem der Ansprüche 1–5, dadurch gekennzeichnet, daß Fragmente von HDL-Antikörpern verwendet werden.

7. Verfahren nach Anspruch 5 oder 6, dadurch gekennzeichnet, daß man Antikörper verwendet, die mit gereinigtem HDL oder gegebenenfalls relipidiertem Apolipoprotein A, C und/oder E als Immunogen erhalten werden.

8. Verfahren nach einem der vorhergehenden Ansprüche, dadurch gekennzeichnet, daß man Antikörper vom Schaf oder Kaninchen verwendet.

9. Verfahren nach einem der vorhergehenden Ansprüche, dadurch gekennzeichnet, daß der Antikörper kovalent an das Polyanion gebunden ist.

10. Reagenz zur Bestimmung der LDL-Fraktion in Körperflüssigkeiten, dadurch gekennzeichnet, daß es den HDL-Antikörper in der Konzentration entsprechend 1 bis 100 mg/ml γ-Globulin, die Polyanionen in einer Konzentration von 0,1–15 g/l und die zweiwertigen Kationen in einer Konzentration von 10–250 mmol/l mit einer Gesamtsalzkonzentration höher als 150 mmol/l enthält.

11. Reagenz nach Anspruch 10, dadurch gekennzeichnet, daß es ein Reagenz zur Bestimmung von Cholesterin enthält.

12. Reagenz nach Anspruch 11, dadurch gekennzeichnet, daß das Reagenz zur Bestimmung von Cholesterin Cholesterinoxidase, ein Cholesterinester spaltendes Enzym oder Enzymsystem, ein System zur Bestimmung von $H_2O_2$ und ein oberflächenaktives Mittel enthält.

13. Reagenz nach Anspruch 12, dadurch gekennzeichnet, daß es im wesentlichen aus HDL-Antikörper, Cholesterinoxidase, Cholesterinesterase, Peroxidase, 3,4-Dichlorphenol, Phenol, 4-Aminophenazon, einem nichtionischen Detergenz, Magnesiumaspartat und Puffer pH 7 bis 8,5 besteht.

14. Reagenz nach einem der Ansprüche 10–13, dadurch gekennzeichnet, daß es die HDL-Antikörper in immobilisierter Form enthält.

15. Reagenz nach einem der Ansprüche 10–14, dadurch gekennzeichnet, daß es HDL-Antikörper enthält, die mit dem Polyanion kovalent verknüpft sind.

## Claims

1. Process for the determination of low density lipoproteins (LDL) in body fluids in which one adds high density lipoprotein (HDL) antibodies to the sample to be investigated, separates off insolubles formed and determines the LDL or one of its components in the supernatant, characterised in that one adds a mixture of a polyanion and a divalent cation to the antibody, whereby the polyanions are present in a concentration of 0.1–15 g/l, the divalent cations in a concentration of 10–250 mmole/l in the reaction mixture with a total salt concentration greater than 150 mmole/l.

2. Process according to claim 1, characterised in that, as polyanions, one adds dextran sulphate, heparin, phosphotungstic acid or polyvinyl sulphate and, as divalent cations, calcium, magnesium or manganese ions.

3. Process according to one of claims 1 or 2, characterised in that the concentration in the reaction mixture of high molecular dextran sulphate amounts to 0.1–8 g/l, in the case of short-chained dextran sulphate to 1–15 g/l, in the case of heparin to 1–15 g/l, in the case of polyvinyl sulphate to 0.2–5 g/l, in the case of phosphotungstic acid to 0.3–6 g/l, in the case of the divalent cations to 10–250 mmole/l.

4. Process according to one of claims 1–3, characterised in that one determines cholesterol as LDL component.

5. Process according to one of claims 1–4, characterised in that the antibody is present in the form of defatted anti HDL antiserum or as purified antibody fraction.

6. Process according to one of claims 1–5, characterised in that fragments of HDL antibodies are used.

7. Process according to claim 5 or 6, characterised in that one uses antibodies which are obtained with purified HDL or possibly relipidated apolipoprotein A, C and/or E as immunogen.

8. Process according to one of the preceding claims, characterised in that one uses antibodies from sheep or rabbits.

9. Process according to one of the preceding claims, characterised in that the antibody is covalently bound to the polyanion.

10. Reagent for the determination of the LDL fraction in body fluids, characterised in that it contains the HDL antibodies in a concentration corresponding to 1 to 100 mg/ml. γ-globulin, the polyanions in a concentration of 0.1–15 g/l and the divalent cations in a concentration of 10–250 mmole/l with a total salt concentration greater than 150 mmole/l.

11. Reagent according to claim 10, characterised in that it contains a reagent for the determination of cholesterol.

12. Reagent according to claim 11, characterised in that the reagent for the determination of cholesterol contains cholesterol oxidase, a cholesterol estersplitting enzyme or enzyme system, a system for the determination of $H_2O_2$ and a surface-active agent.

13. Reagent according to claim 12, characterised in that it consists essentially of HDL antibodies, cholesterol oxidase, cholesterol esterase, peroxidase, 3,4-dichlorophenol, phenol, 4-aminophenazone, a nonionic detergent, magnesium aspartate and buffer pH 7 to 8.5.

14. Reagent according to one of claims 10–13, characterised in that it contains the HDL antibodies in immobilised form.

15. Reagent according to one of claims 10–14, characterised in that it contains HDL antibodies which are covalently linked with the polyanion.

**Revendications**

1. Procédé pour la détermination des lipoprotéines de faible densité (LDL) dans les liquides physiologiques, dans lequel on ajoute à l'échantillon à déterminer de l'anticorps anti-lipoprotéine de haute densité (HDL), on sépare les matières insolubles formées, et dans le surnageant, on détermine les LDL ou un de leurs composants, caractérisé en ce qu'on ajoute à l'anticorps un mélange d'un polyanion et d'un cation bivalent, les polyanions étant présents dans le mélange réactionnel en une concentration de 0,1–15 g/l, et les cations bivalents, en une concentration de 10–250 mmoles/l, avec une concentration totale en sels supérieure à 150 mmoles/l.

2. Procédé selon la revendication 1, caractérisé en ce que comme polyanion on ajoute du sulfate de dextrane, de l'héparine, de l'acide phosphotungstique, ou du sulfate de polyvinyle et comme cation bivalent, on ajoute des ions calcium, des ions magnésium ou des ions manganèse.

3. Procédé selon l'une des revendications 1 ou 2, caractérisé en ce que dans le mélange réactionnel, les concentrations se situent, pour du sulfate de dextrane de masse moléculaire élevée, à 0,1–8 g/l, pour du sulfate de dextrane à courte chaîne, à 1–15 g/l, pour de l'héparine, à 1–15 g/l, pour du sulfate de polyvinyle, à 0,2–5 g/l, pour de l'acide phosphotungstique, à 0,3–6 g/l, pour des cations bivalents, à 10–250 mmoles/l.

4. Procédé selon l'une des revendications 1–3, caractérisé en ce que comme composant des LDL, on détermine le cholestérol.

5. Procédé selon l'une des revendications 1–4, caractérisé en ce que l'anticorps se trouve sous la forme d'antisérum anti-HDL dégraissé ou sous la forme de fraction d'anticorps purifiée.

6. Procédé selon l'une des revendications 1–5, caractérisé en ce que l'on utilise des fragments d'anticorps anti-HDL.

7. Procédé selon la revendication 5 ou 6, caractérisé en ce que l'on utilise des anticorps qui ont été obtenus en tant qu'immunogène avec les HDL purifiées, ou avec des apolipoprotéines A, C et/ou E éventuellement relipidées.

8. Procédé selon l'une des revendications précédentes, caractérisé en ce que l'on utilise des anticorps de mouton ou de lapin.

9. Procédé selon l'une des revendications précédentes, caractérisé en ce que l'anticorps est lié par covalence au polyanion.

10. Réactif pour la détermination de la fraction HDL dans les liquides physiologiques, caractérisé en ce qu'il contient l'anticorps anti-HDL en une concentration correspondant à 1–100 mg/ml de γ-globuline, les polyanions en une concentration de 0,1–15 g/l et les cations bivalents en une concentration de 10–250 mmoles/l, avec une concentration totale en sels supérieure à 150 mmoles/l.

11. Réactif selon la revendication 10, caractérisé en ce qu'il contient un réactif pour la détermination du cholestérol.

12. Réactif selon la revendication 11, caractérisé en ce que le réactif pour la détermination du cholestérol contient de la cholestéroloxydase, une enzyme ou système d'enzyme scindant l'ester de cholestérol, un système pour la détermination de $H_2O_2$ et un agent tensioactif.

13. Réactif selon la revendication 12, caractérisé en ce qu'il est essentiellement constitué d'anticorps anti-HDL, de cholestéroloxydase, de cholestérolesterase, de peroxydase, de 3,4-dichlorophénol, de phénol, de 4-aminophénazone, d'un détergent non ionique d'aspartate de magnésium et de tampon pH 7 à 8,5.

14. Réactif selon l'une des revendications 10–13, caractérisé en ce qu'il contient l'anticorps anti-HDL sous forme immobilisée.

15. Réactif selon l'une des revendications 10–14, caractérisé en ce qu'il contient un anticorps anti-HDL qui est lié par covalence au polyanion.